# EUROPEAN PATENT SPECIFICATION

(11) **EP 1 850 727 B1**
(45) Date of publication and mention of the grant of the patent: **13.04.2011**
(21) Application number: 06720771.2
(22) Date of filing: 15.02.2006
(51) Int. Cl.: A61B 1/07, A61F 9/007, A61B 9/00, G02B 6/00

(54) **HIGH THROUGHPUT ENDO-ILLUMINATOR PROBE**
INNENBELEUCHTUNGSSONDE MIT HOHEM DURCHSATZ
ENDO-ILLUMINATEUR A HAUT RENDEMENT

(30) Priority: 15.02.2005 US 653265 P
(43) Date of publication of application: 07.11.2007
(73) Proprietor: Alcon, Inc., 6331 Hünenberg (CH)
(72) Inventor: SMITH, Ronald T., Newport Coast, CA 92657 (US)
(74) Representative: Hanna, Peter William Derek
(86) International application number: PCT/US2006/005297
(87) International publication number: WO 2006/088938

(56) References cited:
- WO-A-2004/006749
- DE-A1- 3 630 351
- DE-A1- 10 051 057
- US-A- 5 729 643
- US-A- 5 888 194

## Description

The present invention relates generally to surgical instrumentation. In particular, the present invention relates to surgical instruments for illuminating an area during eye surgery. Even more particularly, the present invention relates to a high throughput endo-illuminator probe for illumination of a surgical field.

### BACKGROUND OF THE INVENTION

In ophthalmic surgery, and in particular in vitreo-retinal surgery, it is desirable to use a wide-angle surgical microscope system to view as large a portion of the retina as possible. Wide-angle objective lenses for such microscope systems exist, but they require a wider illumination field than that provided by the cone of illumination of a typical prior-art fiber-optic illuminator probe. As a result, various technologies have been developed to increase the beam spreading of the relatively incoherent light provided by a fiber-optic illuminator. These known wide-angle illuminators can thus illuminate a larger portion of the retina as required by current wide-angle surgical microscope systems. However, these illuminators are subject to an illumination angle vs. luminous flux tradeoff, in which the widest angle probes typically have the least throughput efficiency and the lowest luminous flux (measured in lumens). Therefore, the resultant illuminance (lumens per unit area) of light illuminating the retina is often lower than desired by the ophthalmic surgeon. Furthermore, these wide-angle illuminators typically comprise a larger diameter fiber designed to fit within a smaller gauge (i.e. larger-diameter cannula) probe (e.g., a .0295 inch diameter fiber that will fit within a .0355 inch outer diameter, .0310 inch inner diameter 20 gauge cannula) than the more recent, higher gauge/smaller diameter fiber-optic illuminators necessitated by the small incision sizes currently preferred by ophthalmic surgeons.

Most existing light sources for an ophthalmic illuminator comprise a xenon light source, a halogen light source, or another light source capable of delivering incoherent light through a fiber optic cable. These light sources are typically designed to focus the light they produce into a 20 gauge compatible (e.g..0295 inch diameter) fiber optically coupled to the light source. This is because probes having a 20 gauge compatible optical fiber to transmit light from the light source to a surgical area have been standard for some time. However, the surgical techniques favored by many surgeons today require a smaller incision size and, consequently, higher gauge illuminator probes and smaller diameter optical fibers. In particular, endo-illuminators having a 25 gauge compatible optical fiber are desirable for many small incision ophthalmic procedures. Furthermore, the competing goals of reduced cannula outer diameter (to minimize the size of the incision hole) and maximum fiber diameter (to maximize luminous flux) have typically resulted in the use of very flexible ultrathin-walled cannulas that are not preferred by ophthalmic surgeons. Many ophthalmic surgeons like to use the illumination probe itself to move the eyeball orientation during surgery. An ultra-flexible thin-walled cannula makes it difficult for the surgeon to do this.

Attempts have been made to couple higher gauge optical fiber illuminators to a light source designed to focus light into a 20 gauge compatible optical fiber. For example, one commercially available 25-gauge endo-illuminator probe consists of a contiguous fiber across its 84 inch length. Over most of its length, the fiber has a .020 inch diameter. Near the distal end of the probe, however, the fiber tapers from .020 inch to .017 inch over a span of a few inches and continues downstream from the taper for a few inches at a .017 inch diameter. The fiber numerical aperture ("NA") is 0.50 across its entire length. The fiber NA thus matches the light source beam NA of ~0.5 at its proximal end. This design, however, has at least three disadvantages.

First, the light source lamp is designed to focus light into a 20 gauge compatible fiber with a .0295 inch diameter. The probe's fiber, however, has only a .020 inch diameter. Therefore, a large portion of light from the focused light source beam spot will not enter the smaller diameter fiber and will be lost. Second, due to the fiber diameter tapering from .020 inch to .017 inch, as the transmitted light beam travels through the tapered region its NA increases above 0.50 due to conservation of etendue. However the fiber NA at the distal end remains at 0.5. Therefore, the fiber cannot confine the entire beam within the fiber core downstream of the taper. Instead, a portion of the light source beam (the highest off-axis angle rays) escapes from the core into the cladding surrounding the fiber and is lost. This results in a reduction of throughput of light reaching the distal end of the fiber and emitted into the eye. As a result of these disadvantages, the throughput of the fiber is much less than that of a typical 20 gauge compatible fiber (on average, less than 35% that of the 20 gauge compatible fiber). Third, this probe uses an ultra-thin walled cannula with a .0205 inch outer diameter and a roughly .017 inch inner diameter that has very little stiffness and will flex noticeably when any lateral force is applied to the cannula.

Another commercially available 25-gauge endo-illuminator probe consists of a contiguous, untapered .0157 inch diameter fiber having an NA of 0.38. Like the tapered prior art endo-illuminator described above, this untapered design has a fiber throughput that is much less than that of a typical 20 gauge compatible fiber. This is because, again, the light source lamp is designed to focus light into a 20 gauge compatible, .0295 inch diameter, fiber. Therefore, a large portion of light from the focused light source beam spot will not enter the .157 inch diameter fiber and will be lost. Also, the fiber NA of 0.38 is much less than the light source beam NA of 0.50. Therefore, a large portion of the light that is focused into the fiber will not propagate through the fiber core and will instead escape the core and pass into the cladding and be lost. Combined, these two disadvantages result in a fiber throughput that is on average less than 25% that of a typical 20 gauge compatible fiber. Furthermore, this probe also uses an ultra-thin walled cannula with a .0205 inch outer diameter and a roughly .017 inch inner diameter that has very little stiffness and will flex noticeably when any lateral force is applied to the cannula.

In another arrangement WO2004/006749 describes a light transmitting apparatus comprised of a single length of optic fiber having opposite proximal and distal ends with a light source connector at the fiber proximal end and the distal end counted to an ophthalmic surgery instrument. The fiber is tapered adjacent the optical fiber proximal end and is shaped complementary to the conical interior of surface of the bore of the light source connector.

A further disadvantage of prior art small-gauge (e.g., 2 5 gauge) illuminators is that they are typically designed to emit transmitted light over a small angular cone (e.g., ~30 degree half angle and ~22 degree half angle, respectively, for the two prior art examples above). Ophthalmic surgeons, however, prefer to have a wider angular illumination pattern to illuminate a larger portion of the retina.

Therefore, a need exists for a high throughput endo-illuminator that can reduce or eliminate the problems associated with prior art high-gauge endo-illuminators, particularly the problems of matching a fiber proximal cross-section to a light source focused spot size while having a fiber NA higher than the light source beam NA throughout the length of the fiber, of emitting the transmitted light source light over a small angular cone, and of having ultra-thin walled, overly flexible cannulas.

### BRIEF SUMMARY OF THE INVENTION

The embodiments of the high throughput endo-illuminator of the present invention substantially meet these needs and others. The invention provides an illuminator in accordance with claim 1. Advantageous features are provided in accordance with the dependent claims. One embodiment of this invention is a high throughput illumination surgical system comprising: a light source for providing a light beam; a first proximal optical fiber, optically coupled to the light source for receiving and transmitting the light beam; a second distal optical fiber, distinct from the first optical fiber optimally coupled to a distal end of the proximal optical fiber, for receiving the light beam and emitting the light beam to illuminate a surgical site, wherein the distal optical fiber comprises a tapered section having a proximal-end diameter larger than a distal-end diameter; a handpiece, operably coupled to the distal optical fiber; and a cannula, operably coupled to the handpiece, for housing and directing the distal optical fiber.

The tapered section's proximal end diameter can be the same as the diameter of the proximal optical fiber, and can be, for example, a 20 gauge compatible diameter. The tapered section's distal end diameter can be, for example, a 25 gauge compatible diameter. The cannula can be a 25 gauge innerdiameter cannula. The proximal optical fiber can preferably have an NA equal to or greater than the NA of the light source beam and the distal optical, fiber preferably can have an NA greater than that of the proximal optical fiber and greater than that of the light source beam at any point in the distal optical fiber (since the light beam NA can increase as it travels through the tapered section).

The distal optical fiber can be a higher-gauge (e.g., 25 gauge compatible) optical fiber with the distal end of the distal optical fiber co-incident with the distal end of the cannula. The distal optical fiber can also be coupled to the cannula so that the distal end of the distal optical fiber extends past the cannula distal end by approximately .005 inches. The cannula and the handpiece can be fabricated from biocompatible materials. The optical cable can comprise a proximal optical fiber, a first optical connector operably coupled to the light source and a second optical connector operably coupled to the handpiece (or other means of optically coupling the proximal optical fiber to the distal optical fiber). Alternatively, the handpiece and optical cable can be operably coupled by any other means known to those in the art. The optical connectors can be SMA optical fiber connectors. The distal optical fiber and proximal optical fiber are optically coupled and, at the coupling interface, can be of a compatible gauge so as to more efficiently transmit the light beam from the light source to the surgical field. For example, both fibers can be of equal gauge at the coupling point.

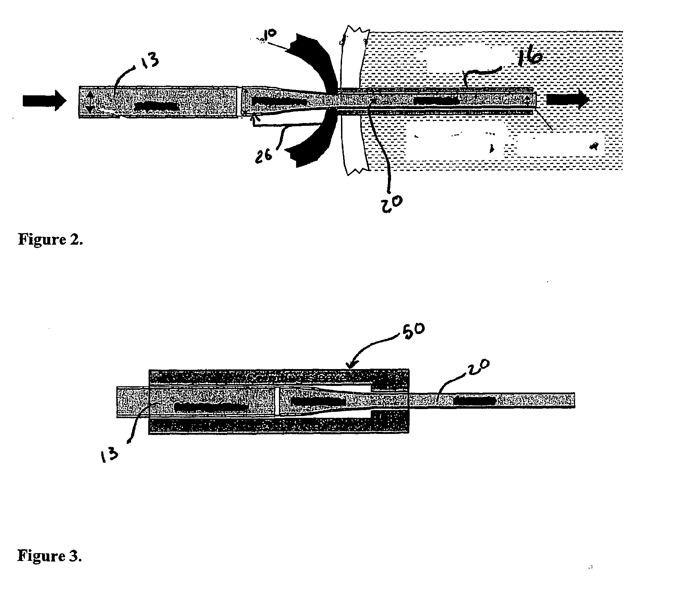

As shown in FIGURE 2, the proximal optical fiber can be a larger diameter optical fiber (e.g., 20 gauge compatible) operable to be optically coupled to the light source to receive light from the light source. The distal optical fiber can be a high numerical aperture ("NA"), smaller diameter (e.g., 25 gauge compatible) optical fiber or cylindrical light pipe located downstream of the proximal optical fiber, comprising a high NA tapered section. The tapered section can be tapered so as to have a diameter that matches the proximal optical fiber diameter at the point of optical coupling (e.g., the tapered section starts at .0295 inches—20 gauge compatible-- where it couples to the proximal optical fiber and tapers to .015 inches -- 25 gauge compatible -- downstream of the coupling point). In another embodiment, the tapered section can be a separate section that optically joins the proximal optical fiber and the distal optical fiber, tapering from the diameter of the first to the diameter of the second over its length.

To enable additional advantages of the embodiments of this invention, the distal optical fiber can be operably coupled to the handpiece to enable linear displacement of the optical fiber within the cannula. The distal end of the distal optical fiber can then move relative to an open aperture of the cannula, such that it can extend beyond the cannula aperture. The handpiece can include a means, such as a push/pull mechanism, for adjusting the linear displacement of the distal optical fiber. Other adjusting means as known to those in the art can also be used. Adjusting the linear displacement of the distal optical fiber will change the amount of the distal optical fiber that extends beyond the cannula aperture and can, in some instances, change the angle of the scattered light from the distal optical fiber end. Thus, by adjusting the linear displacement of the distal optical fiber, the angle of illumination and the amount of illumination provided by the distal optical fiber to illuminate the surgical field (e.g., the retina of an eye) can be adjusted by the surgeon.

Other embodiments of the present invention can include a surgical handpiece embodiment of the high throughput endo-illuminator of the present invention for use in ophthalmic surgery. Further, embodiments of this invention can be incorporated within a surgical machine or system for use in ophthalmic or other surgery. Other uses for a high throughput illuminator designed in accordance with the teachings of this invention will be known to those familiar with the art.

### BRIEF DESCRIPTION OF THE SEVERAL VIEWS OF THE DRAWINGS

A more complete understanding of the present invention and the advantages thereof may be acquired by referring to the following description, taken in conjunction with the accompanying drawings, in which like reference numbers indicate like features and wherein:
FIGURE 1 is a simplified diagram of one embodiment of a high throughput endo-illumination system in accordance with the teachings of this invention;
FIGURE 2 is a close-up view of one embodiment of a high throughput endoilluminator of the present invention;
FIGURE 3 is a diagram showing a coupling sleeve for aligning optical fibers in accordance with this invention;
FIGURE 4 is a diagram illustrating a system for creating a belled optical fiber in accordance with this invention;
FIGURE 5a is a diagram illustrating a cannula-assisted belling process in accordance with this invention;
FIGURE 5b is a photograph of an optical fiber with a typical cannula-assisted bell produced according to the process of FIGURE 5a;
FIGURE 6 is a diagram illustrating a method of bonding a belled fiber in a cannula in accordance with this invention;
FIGURE 7 is a diagram illustrating a system for molding a belled fiber in accordance with this invention;
FIGURE 8 is a diagram illustrating a system for creating a stretched and belled optical fiber in accordance with this invention;
FIGURE 9 is a diagram illustrating another embodiment of the high throughput endo-illuminator of this invention having a separate tapered section;
FIGURE 10 is a is a diagram showing a coupling sleeve for aligning optical fibers and a separate tapered section according to one embodiment of the present invention;
FIGURE 11 is a diagram illustrating another embodiment of the high throughput endo-illuminator of this invention having a distal light pipe;
FIGURE 12 is a diagram illustrating the use of one embodiment of the high throughput endo-illuminator of this invention in an ophthalmic surgery;
FIGURE 13 is a diagram illustrating an embodiment of an adjusting means 40 in accordance with the present invention; and
FIGUREs 14 and 15 show exemplary embodiments of a contiguous optical fiber endo-illuminator in accordance with this invention.

### DETAILED DESCRIPTION OF THE INVENTION

Preferred embodiments of the present invention are illustrated in the FIGURES, like numerals being used to refer to like and corresponding parts of the various drawings.

The various embodiments of the present invention provide for a higher gauge (e.g., 20 and/or 25 gauge compatible) optical fiber based endo-illuminator device for use in surgical procedures, such as in vitreo-retinal/posterior segment surgery. Embodiments of this invention can comprise a handpiece, such as the Alcon-Grieshaber Revolution-DSP^{™} handpiece sold by Alcon Laboratories, Inc., of Fort Worth, Texas, operably coupled to a cannula, such as a 25 gauge cannula. The inner dimension of the cannula can be used to house a distal optical fiber, tapered in accordance with the teachings of this invention. Embodiments of the high throughput endo-illuminator can be configured for use in the general field of ophthalmic surgery. However, it is contemplated and it will be realized by those skilled in the art that the scope of the present invention is not limited to ophthalmology, but may be applied generally to other areas of surgery where high throughput, higher gauge illumination may be required.

An embodiment of the high throughput endo-illuminator of this invention can comprise a distal optical fiber, stem (cannula) and a handpiece fabricated from biocompatible polymeric materials, such that the invasive portion of the illuminator is a disposable surgical item. Unlike the prior art, the embodiments of the endoilluminator of this invention can provide high optical transmission/high brightness with low optical losses. Embodiments of this invention fabricated from biocompatible polymeric materials can be integrated into a low cost, articulated handpiece mechanism, such that these embodiments can comprise an inexpensive disposable illuminator instrument.

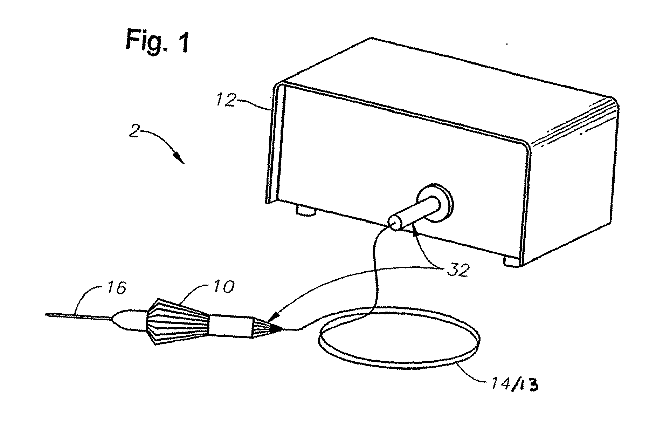

FIGURE 1 is a simplified diagram of a surgical system 2 comprising a handpiece 10 for delivering a beam of relatively incoherent light from a light source 12 through cable 14 to the distal end of a stem (cannula) 16. Cable 14 can comprise a proximal optical fiber 13 of any gauge fiber optic cable as known in the art, but proximal optical fiber 13 is preferably a 20 or 25 gauge compatible fiber. Stem 16 is configured to house a distal optical fiber 20, as is more clearly illustrated in FIGUREs 2-11. Coupling system 32 can comprise an optical fiber connector at the proximal end of optical cable 14 to optically couple light source 12 to proximal optical fiber 13 within optical cable 14.

FIGURE 2 is a close-up view of one embodiment of a high throughput endoilluminator of the present invention, including handpiece 10, cannula 16 and their respective internal configurations. Stem 16 is shown housing a non-tapered distal section of distal optical fiber 20. Distal optical fiber 20 is optically coupled to proximal optical fiber 13, which is itself optically coupled to light source 12 to receive light from the light source 12. Proximal optical fiber 13 can be a larger diameter, small NA (e.g., .5 NA) optical fiber, such as a 20 gauge compatible optical fiber. Distal optical fiber 20 can be a high numerical aperture ("NA"), smaller diameter (e.g., 25 gauge compatible) optical fiber or cylindrical light pipe located downstream of the proximal optical fiber. Distal optical fiber 20 can comprise a high NA tapered section 26, wherein the diameter of the upstream end of distal optical fiber 20 matches the proximal optical fiber 13 diameter at the point of optical coupling (e.g., the distal optical fiber 20 diameter is .0295 inches — 20 gauge compatible — where it couples to the proximal optical fiber 13) and tapers to, for example, .015 inches — 25 gauge compatible, downstream of the coupling point through tapered section 26. In another embodiment, the tapered section 26 can be a separate optical section that optically couples proximal optical fiber 13 and distal optical fiber 20, tapering from the diameter of the first to the diameter of the second over its length. Tapered section 26 can be made of optical grade machined or injection-molded plastic or other polymer.

Handpiece 10 can be any surgical handpiece as known in the art, such as the Revolution-DSP^{™} handpiece sold by Alcon Laboratories, Inc. of Fort Worth, Texas. Light source 12 can be a xenon light source, a halogen light source, or any other light source capable of delivering incoherent light through a fiber optic cable. Stem 16 can be a small diameter cannula, such as a 25 gauge cannula, as known to those in the art. Stem 16 can be stainless steel or a suitable biocompatible polymer (e.g., PEEK, polyimide, etc.) as known to those in the art.

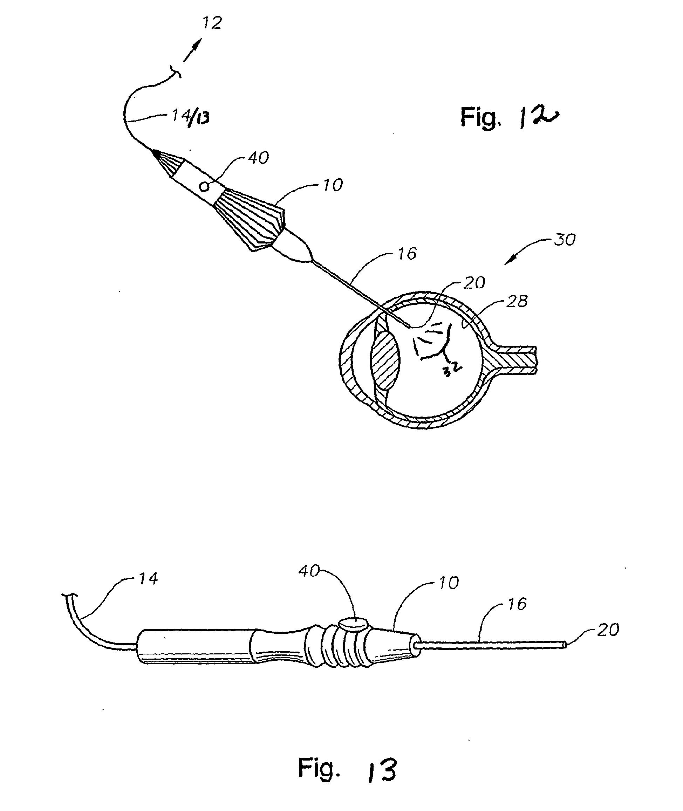

The proximal optical fiber 13, distal optical fiber 20 and/or stem 16 can be operably coupled to the handpiece 10, for example, via an adjusting means 40, as shown in FIGUREs 12 and 13. Adjusting means 40 can comprise, for example, a simple push/pull mechanism as known to those in the art. Light source 12 can be operably coupled to handpiece 10 (i.e., optically coupled to proximal optical fiber 13 within optical cable 14) using, for example, standard SMA (Scale Manufacturers Association) optical fiber connectors at the proximal end of fiber optic cable 14. This allows for the efficient transmission of light from the light source 12 to a surgical site through proximal optical fiber 13, passing within handpiece 10, through tapered section 26 (whether separate or integral to distal optical fiber 20) and optical fiber 20 to emanate from the distal end of distal optical fiber 20 and stem 16. Light source 12 may comprise filters, as known to those skilled in the art, to reduce the damaging thermal effects of absorbed infrared radiation originating at the light source. The light source 12 filter(s) can be used to selectively illuminate a surgical field with different colors of light, such as to excite a surgical dye.

The embodiment of the high throughput endo-illuminator of this invention illustrated in FIGURE 2 comprises a low-NA, larger diameter proximal optical fiber 13 optically coupled to a tapered, high-NA, smaller diameter distal optical fiber 20. The proximal optical fiber 13 (the upstream fiber) can be a 0.50 NA plastic fiber (e.g., to match the NA of the light source 12), having a polymethyl methacrylate (PMMA) core and a 0.030" (750 micron) core diameter, or other such comparable fiber as known to those having skill in the art. For example, such a fiber is compatible with the dimensions of the focused light spot from a 20 gauge light source 12, such as the ACCURUS^{®} illuminator manufactured by Alcon Laboratories, Inc. of Fort Worth, Texas. For example, suitable fibers for the proximal optical fiber 13 of the embodiments of this invention are produced by Mitsubishi (Super-Eska fiber), which can be purchased through Industrial Fiber Optics, and Toray, which can be purchased through Moritex Corporation.

Suitable fibers for the distal optical fiber 20 (downstream fiber) are Polymicro's High OH (FSU), 0.66 NA, silica core/Teflon AF clad optical fiber, having a core diameter that can be custom-made to required specifications and Toray's PJU-FB500 0.63 NA fiber (486 micron core diameter). Regardless of the material chosen for the distal optical fiber 20, in one embodiment of this invention a tapered section 26 must be created in distal optical fiber 20 in accordance with the teachings above. Methods of creating a taper in, for example, the proximal end of distal optical fiber 20 include (1) belling the fiber, and (2) stretching the fiber. In another embodiment, tapered section 26 can be a separate optical section; for example, tapered section 26 can be an acrylic taper created by diamond turning or injection molding. Once tapered section 26 is created in distal optical fiber 20, the different sections can be assembled in a completed illuminator probe. For example, the optical fibers (and tapered section 26, in some embodiments) can be bonded together with optical adhesive to hold the optical elements together and to eliminate Fresnel reflection losses between them. The optical elements can be assembled by precision alignment using an x-y-z motion stage and a video microscope. Alternatively, the optical elements can be assembled with the aid of a coupling sleeve 50, for example, as shown in FIGURE 3, that forces the optical elements into translational and angular alignment.

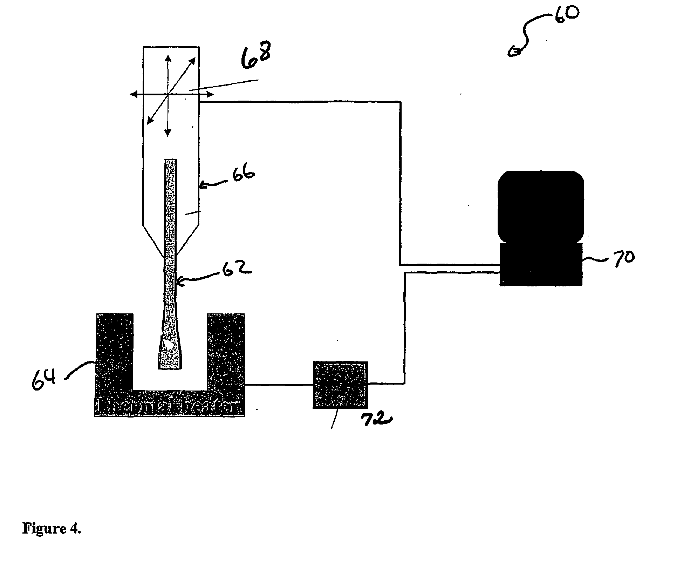

Belling an optical fiber comprises heating an end of the optical fiber at a high temperature for a short time (e.g., a few seconds) until the end "bells" or flares into an expanded diameter. FIGURE 4 shows a system 60 for belling an optical fiber. Typically, optical fibers are created by pulling a softened large diameter cylinder of core material into a long, small diameter fiber. The pulled fiber is then allowed to resolidify. The resulting fiber tends to have stored within it compressive forces that are unleashed when the fiber is reheated to the softening point. In addition, fibers provided in specific standard diameters (e.g., 0.020") by a fiber vendor may need to be stretched further in order to attain a desired diameter (e.g., 0.015 - 0.017" for 25 gauge endo-illuminators). This stretching can add further compressive forces to the fiber.

When a fiber 62 (which can be formed into a distal optical fiber 20 of FIGURE 2) is inserted into a thermal heater 64 cavity as in FIGURE 4 and heated to its softening point, the fiber 62 shrinks in length in response to the compressive forces that are unleashed. Because the volume of the fiber 62 is fixed, shrinking in length results in an increase in diameter. In practice, there is typically a gradual, S-shaped taper transition between the wide entrance diameter and the narrow diameter of the resulting fiber 62. One way to create a belled fiber 62 in a repeatable manner is to insert the fiber 62 into a fiber chuck 66 that is attached to a computer-controlled x-y-z translation stage 68. A processor (computer) 70 can control the vertical (z-axis) insertion speed, insertion depth, dwell time, and retraction speed of the translator 68 as well as the temperature of the thermal heater via temperature controller 72. This type of belling process is effective for belling plastic fibers 62.

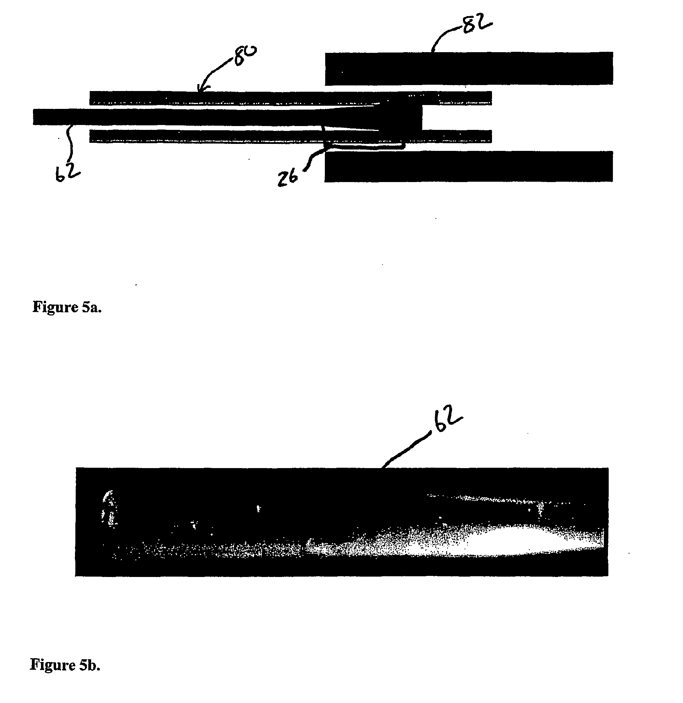

Belling of an optical fiber 62 can also be accomplished by a process of cannula-assisted belling. FIGURE 5a illustrates a cannula-assisted belling process in which the optical fiber 62 is inserted into a cannula 80 and the cannula 80 and fiber 62 are then inserted into a thermal heater 82 cavity. As the fiber 62 bells within the cannula 82, its shape and size are restricted by the cannula 82 to obtain various performance advantages. For example, the diameter of the resulting bell will match the inner diameter of the cannula 82. Thus, by adjusting the cannula 82 inner diameter, the resultant bell diameter can be made to match the diameter of a proximal optical fiber 13 to which the belled fiber 62 can be optically coupled in the manner described with reference to FIGURE 2. The photopic throughput of an illuminator probe incorporating such matched fibers will be increased over that of prior art illuminators. Further, the resultant bell is long relative to its width and has a gradual taper, the bell axis is essentially parallel to the axis of the unbelled fiber 62, the proximal end face of the bell is flat and is nearly normal to the optical axis of the fiber 62, and the side surface of the bell is optically smooth and glossy. Each of these attributes is desirable to enhance optical performance.

FIGURE 5b is a photograph of a fiber 62 with a typical cannula-assisted bell.

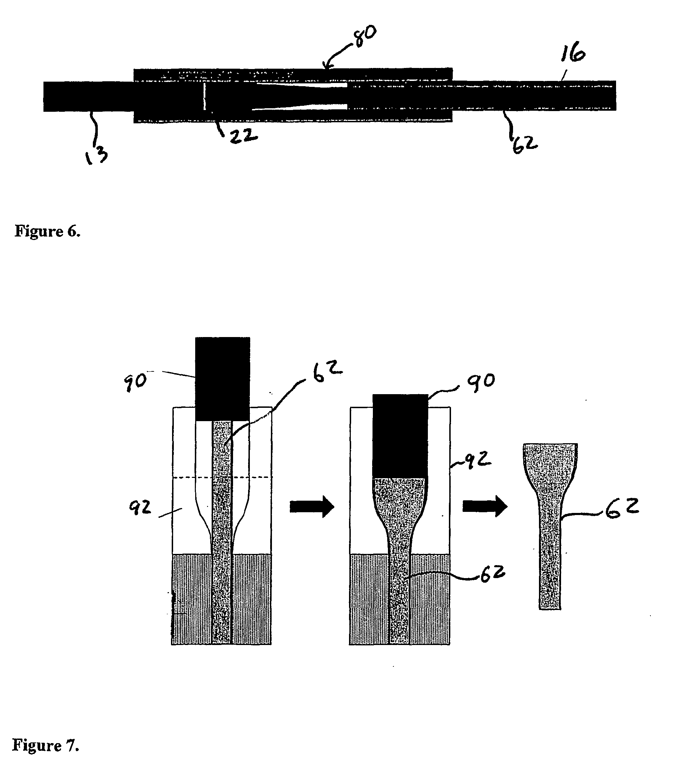

As a further advantage of cannula-assisted belling, when a fiber 62 has been recessed within the cannula 80 to form the bell (tapered section 26), it is possible to bond the belled fiber 62 to a larger diameter, proximal optical fiber 13 (e.g., 20 gauge compatible, 0.5 NA fiber) without having to remove the belled fiber 62 from the cannula 80. FIGURE 6 illustrates one such method of bonding a belled fiber 62 (distal optical fiber 20) to a proximal optical fiber 13 with an optical adhesive 22 within a cannula 80. Optical adhesive 22 can be any index-matching optical-grade adhesive as will be known to those having skill in the art, such as Dymax 142-M optical adhesive Belled fiber 62/distal optical fiber 20 can be operably coupled (bonded) to a, for example, 25 gauge cannula/stem 16 which can in turn be crimped within a 20-gauge cannula 80.

Molding is another process by which a tapered section 26 can be formed in an optical fiber 62. FIGURE 7 illustrates a molding technique in which a bell is formed in a fiber 62 by heating one end of fiber 62 to its softening point and using a piston 90 to push it into a mold 92 cavity that forces the fiber 62 end to assume a bell shape. Molding may potentially be used to shape plastic and glass fibers 62.

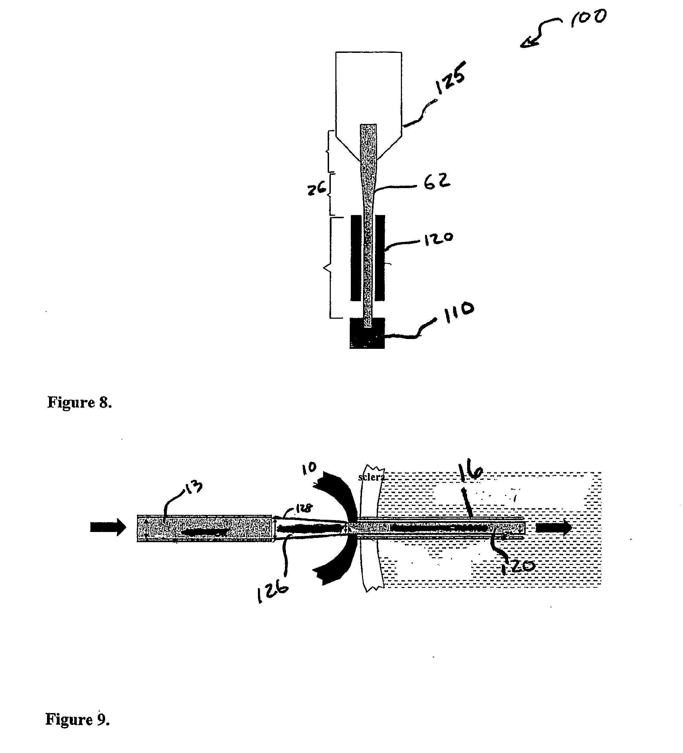

Still another technique for forming a tapered section 26 in an optical fiber 62 is stretching of the optical fiber 62. FIGURE 8 illustrates one system 100 for forming a stretched optical fiber 62. Stretching a fiber 62 is accomplished by attaching a weight 110 to a vertical plastic or glass fiber 62 that is suspended within a cylindrical heater 120 from a chuck 125. Within heater 120, the fiber 62 softens and then stretches to a smaller diameter due to the action of the weight 110. The portion of fiber 62 attached to the fiber chuck 125 remains unheated and therefore retains its original larger diameter. The portion of fiber 62 between fiber chuck 125 and the heater 120 is stretched into a tapered transition section 26. The length of tapered section 26 can be adjusted by controlling how rapidly the temperature transitions along the fiber 62.

The methods described above can be combined to produce a desired distal optical fiber 20 that may have better properties than if only one method were used. For example, a standard .020 inch core diameter fiber 62 can be stretched so that its distal end will fit into a .015 inch - .017 inch (e.g., 25 gauge) inner diameter cannula 16. The proximal end can then be belled to a .0295 inch core diameter to match the core diameter of a typical 20 gauge compatible, 0.5 NA proximal optical fiber 13.

Once a tapered section 26 has been added to an optical fiber 62 to form a distal optical fiber 20, the distal optical fiber 20 and the proximal optical fiber 13 can be optically coupled by, for example, precision alignment with a video microscope and x-y-z translator, or preferably, with a coupling sleeve 50 of FIGURE 3. Proximal optical fiber 13 and distal optical fiber 20 can be coupled together using Dymax 142-M optical adhesive 22, which rapidly cures upon exposure to ultraviolet or low wavelength visible light, or another comparable index-matching optical adhesive 22 as will be known to those having skill in the art. Proximal optical fiber 13 and distal optical fiber 20 can be assembled into a high-throughput endo-illuminator probe in accordance with the present invention, in one embodiment, as follows:
- Insert the narrow end of the distal optical fiber 20 into the large diameter hole of the coupling sleeve 50.
- Slide the distal optical fiber 20 through the coupling sleeve 50 so that the narrow end of the distal optical fiber 20 passes through the narrow downstream hole of the coupling sleeve 50.
- Continue to slide the distal optical fiber 20 into the coupling sleeve 50 until the tapered section 26 contacts the narrow downstream hole of the coupling sleeve 50 and can slide no further.
- Place a small amount of adhesive 22, effective to bond the distal optical fiber 20 and proximal optical fiber 13, onto the distal end of a proximal optical fiber 13.
- Insert the adhesive covered distal end of proximal optical fiber 13 into the large diameter opening of the coupling sleeve 50.
- Slide the proximal optical fiber 13 into the coupling sleeve 50 until the adhesive 22 makes contact with the large diameter end of distal optical fiber 20. Apply light pressure to the proximal optical fiber 13 to push it against the distal optical fiber 20 within the coupling sleeve 50 such that the adhesive line between the two fibers 13/20 is pushed thin and extends into the optical fiber/coupling sleeve 50 interface region.
- Connect the proximal end of the proximal optical fiber 13 to an illuminator, such as the ACCURUS^{®} white light illuminator, and activate the illuminator to flood the adhesive with light until the adhesive is cured. With the ACCURUS^{®} illuminator on HI 3 setting, typically only 10 - 60 seconds of light curing is required.
- For added mechanical strength, adhesive 22 can optionally be applied to the joint between the proximal optical fiber 13 and the upstream end of the coupling sleeve 50 and to the joint between the distal optical fiber 20 and the downstream end of the coupling sleeve 50 and cured with ultraviolet or low wavelength visible light.
- A cannula 16 and handpiece 10 can be attached in any manner known to those skilled in the art to yield a completed 25 gauge endo-illuminator in accordance with this invention.

Another embodiment of the high throughput endo-illuminator of this invention is illustrated in FIGURE 9. The embodiment of FIGURE 9 comprises a low-NA, larger diameter proximal optical fiber 13 optically coupled to a high-NA, smaller diameter distal optical fiber 120 by a separate high-NA plastic or glass tapered section 126. Tapered section 126 in this embodiment is a separate optical element joining the proximal and distal optical fibers 13/20. In an exemplary implementation, optical adhesive 22, such as Dymax 142-M, can be used to join the three elements together.

The proximal optical fiber 13 (the upstream fiber) can be a 0.50 NA plastic fiber (e.g., to match the NA of the light source 12), having a polymethyl methacrylate (PMMA) core and a 0.030" (750 micron) core diameter, or other such comparable fiber as known to those having skill in the art. As in the first embodiment of this invention, such a proximal optical fiber 13 is compatible with the dimensions of the focused light spot from a 20 gauge light source 12, such as the ACCURUS^{®} illuminator. Suitable fibers for the distal optical fiber 20 (downstream fiber) are Polymicro's High OH (FSU), 0.66 NA, silica core/Teflon AF clad optical fiber, having a core diameter that can be custom-made to required specifications and Toray's PJU-FB500 0.63 NA fiber (486 micron core diameter).

Tapered section 126 of this embodiment can be fabricated by diamond turning, casting, or injection molding. For example, tapered section 126 can comprise a diamond-turned acrylic optical section. Tapered section 126 is unlike an optical fiber (e.g., proximal optical fiber 13) in that is has no cladding. Because it is a stand-alone material, tapered section 126 has an NA dependent on the refractive index of the taper and the refractive index of a surrounding medium. If the tapered section 126 is designed to reside within the handpiece 10 so that it is not exposed to liquid, such as saline solution from within an eye, then the medium surrounding the tapered section 126 is contemplated to be air, and the NA of tapered section 126 will be essentially 1. This NA is much greater than the NA of the light beam passing through the tapered section 126; therefore, the transmittance of light through tapered section 126 can theoretically be as high as 100%.

If an embodiment of the endo-illuminator of this invention is designed so that the tapered section 126 is exposed to an ambient medium other than air, such as saline solution, optical adhesive, or plastic hand piece material, etc., the tapered section 126 can be prevented from spilling light into the ambient medium by coating a layer 128 of low refractive index material on the outside surface of tapered section 126. For example, Teflon has a refractive index of 1.29. - 1.31. If the tapered section 126 outer surface is coated with Teflon, the resulting tapered section 126 NA will be 0.71 - 0.75, and most of the light transmitted within the tapered section 126 can be prevented from escaping into the surrounding medium. In other embodiments, portions of the tapered section 126 surface that may come into contact with a non-air ambient medium can instead be coated with a reflective metal or dielectric coating to keep transmitted light confined within the tapered section 126.

The embodiment shown in FIGURE 9, comprising, for example, a 100 inch long .0295 inch core diameter, 0.5 NA proximal optical fiber 13, a 37 mm, .0165 inch diameter, 0.66 NA distal optical fiber 20 and a .0295 inch to .0146 inch, over a .25 inch length, acrylic tapered section 126, can have an average transmittance of 46.5% (standard deviation of 3.0%) relative to a 20 gauge compatible optical fiber. This transmittance is much better than that of prior art illuminators having, for example, an average transmittance below 35% and 25%, respectively, for the prior art examples previously described.

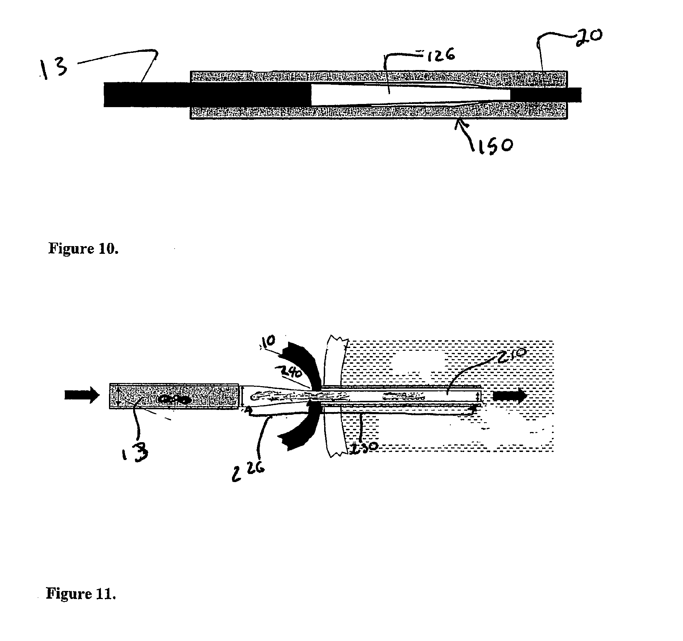

The embodiment of the present invention shown in FIGURE 9 can be assembled using precision alignment with a video microscope and an x-y-z translation stage or using a coupling sleeve 150, such as shown in FIGURE 10. The proximal and distal optical fibers 13 and 20 can be plastic or glass, although in the example of FIGURE 9 proximal optical fiber 13 is a plastic fiber and distal optical fiber 20 is a glass fiber. Proximal optical fiber 13, tapered section 126 and distal optical fiber 20 can be coupled together using Dymax 142-M optical adhesive, which rapidly cures upon exposure to ultraviolet or low wavelength visible light, or another comparable index-matching optical adhesive 22 as will be known to those having skill in the art. Proximal optical fiber 13, tapered section 126 and distal optical fiber 20 can be assembled into a high-throughput endo-illuminator probe in accordance with the present invention, in this embodiment, as follows:
- Insert the narrow end of tapered section 126 into the large diameter opening of coupling sleeve 150.
- Slide tapered section 126 through coupling sleeve 150 until it contacts the narrow downstream inner wall of the coupling sleeve 150 and can go no further.
- Place a small amount of adhesive 22, effective to bond the proximal optical fiber 13 and the tapered section 26, onto the onto the distal end of the proximal optical fiber 13.
- Insert the adhesive covered distal end of proximal optical fiber 13 into the large diameter opening of coupling sleeve 150.
- Slide the proximal optical fiber 13 into coupling sleeve 150 until the adhesive 22 makes optical contact with the tapered section 126. Apply light pressure to the proximal optical fiber 13 to push it against the tapered section 126 within the coupling sleeve 150 such that the adhesive line between the two is pushed thin.
- Connect the proximal end of the proximal optical fiber 13 to an illuminator, such as the ACCURUS^{®} white light illuminator, and activate the illuminator to flood the adhesive with light until the adhesive is cured. With the ACCURUS^{®} illuminator on HI 3 setting, typically only 10 - 60 seconds of light curing is required.
- For added mechanical strength, adhesive 22 can optionally be applied to the joint between the proximal optical fiber 13 and the upstream end of the coupling sleeve 150 and cured with ultraviolet or low wavelength visible light.
- Place a small amount of adhesive 22, effective to bond the distal optical fiber 20 and tapered section 126 to one another, onto the proximal end of the distal optical fiber.
- Insert the adhesive covered proximal end of distal optical fiber 20 into the small diameter opening of the coupling sleeve 150.
- Slide the distal optical fiber 20 into the coupling sleeve 150 until the adhesive 22 makes optical contact with the distal end of tapered section 126. Apply light pressure to the distal optical fiber 20 to push it against the tapered section 126 within the coupling sleeve 150 such that the adhesive line between the two is pushed thin.
- Connect the proximal end of the proximal optical fiber 13 to an illuminator, such as the ACCURUS^{®} white light illuminator, and activate the illuminator to flood the adhesive with light until the adhesive is cured. With the ACCURUS^{®} illuminator on HI 3 setting, typically only 10 - 60 seconds of light curing is required.
- For added mechanical strength, adhesive 22 can optionally be applied to the joint between the distal optical fiber 20 and the downstream end of the coupling sleeve 150 and cured with ultraviolet or low wavelength visible light.
- A cannula 16 and handpiece 10 can be attached in any manner known to those skilled in the art to yield a completed 25 gauge endo-illuminator in accordance with this invention.

FIGURE 11 shows an embodiment of the high throughput endo-illuminator of this invention comprising a low-NA, larger diameter proximal optical fiber 13 optically coupled to a high-NA, light pipe 210 comprising a tapered section 226 and a straight section 230. Light pipe 210 can be made of plastic or glass and can be fabricated using diamond turning, casting, or injection molding. When made of acrylic, the NA of the acrylic/saline interface is 0.61 and the acceptance angular bandwidth of the light pipe 210 will be 38 degrees, which is significantly higher that the angular bandwidth of existing illuminator probes. The throughput of this embodiment of the illuminator probe of this invention will thus be significantly higher than the throughput of prior art probes.

To prevent transmitted light within light pipe 210 from spilling out at a light pipe/handpiece interface, that region on the surface of the light pipe 210 can be coated with Teflon or a reflective metallic or dielectric coating 240. Alternatively, the entire distal end of the light pipe 210 (from the pipe/handpiece interface to the distal end) can be coated with Teflon. Since Teflon has a refractive index of 1.29 - 1.31, the resultant NA of the acrylic light pipe 210 would be 0.71 - 0.75 and the half angle of the angular bandwidth would be 45 -- 49 degrees, resulting in significantly higher throughput than prior art probes.

Embodiments of the present invention provide a high throughput endoilluminator that, unlike the prior art, successfully matches an optical fiber path, at a proximal end, to a light source focused spot size while having a fiber NA higher than the light source beam NA throughout the length of the fiber. Further, embodiments of this invention can emit the transmitted light source light over a larger angular cone (provide a wider field of view) than prior art higher gauge illuminators. Embodiments of this invention can comprise 25 gauge endo-illuminator probes, 25 gauge wide-angle endo-illuminator probes (with the addition of a sapphire lens, bulk diffuser, diffraction grating, or some other angle dispersing element at the distal end of the probe such as in co-owned U.S. Patents No 2005078910, 2005075628, 2007255264, US2007100326 and 2007100327), chandelier probes, as known to those skilled in the art (with removal of the cannula 16, shortening of the distal length, and minor modifications to the distal end of the probe), and/or a variety of other ophthalmic endo-illumination devices as may be familiar to those having skill in the art, having higher throughput than prior art probes.

Embodiments of the present invention can comprise a tapered section 26/126/226 having a larger angular acceptance bandwidth than an upstream proximal optical fiber 13 (i.e., the tapered section 26 has a higher NA). Furthermore the NA of the tapered section 26/126/226 is higher than the NA of the light beam passing through it. Therefore, transmitted light passing through the tapered section 26/126/226 from a larger diameter proximal optical fiber 13 to a smaller diameter distal optical fiber 20 is transmitted with high efficiency. In passing through the tapered section 26/126/226, a light beam is forced into a smaller diameter. Therefore, as a consequence of conservation of etendue, the resultant angular spread of the light beam (i.e., the beam NA) must increase. Also, the smaller diameter distal optical fiber 20 downstream from the tapered section 26/126/226 has a high fiber NA that is equal to or greater than the beam NA. This insures high transmittance propagation through the core of the distal optical fiber 20 to its distal end where it can be emitted into an eye.

The embodiments of the present invention thus have various advantages over the prior art, including higher throughput. The proximal end of optical fiber path is designed to match the focused spot size of an illuminator lamp 12 (e.g., .0295 inch), yielding increased light injected into the fiber. The NA of the tapered section 26/126/226 is higher than the beam NA so the transmittance of light across the tapered section 26 can be as high as 100%. Also, the NA of the distal optical fiber 20 is high (e.g. 0.66 NA for a Polymicro glass fiber), to ensure that that more of the downstream light will remain within the core of the distal optical fiber 20 and less light will escape into the cladding and be lost.

Another advantage of the embodiments of the present invention is a wider angular coverage than prior art illuminators. Current 25 gauge illuminators are designed to spread light over a small angular cone. However, ophthalmic surgeons would prefer to have a wider angular illumination pattern so they can illuminate a larger portion of the retina. One aspect of the embodiments of this invention is that the emitted light beam angular spread increases as a result of the tapered section 26/126/226 and the distal optical fiber 20 has a high acceptance angular bandwidth (i.e., higher NA) in order to transmit this light down the core. As a result, the emitted light cone has a higher angular spread.

FIGURE 12 illustrates the use of one embodiment of the high throughput endo-illuminator of this invention in an ophthalmic surgery. In operation, handpiece 10 delivers a beam of incoherent light through stem 16 (via proximal optical fiber 13 and distal optical fiber 20/tapered section 26/126/226) to illuminate a retina 28 of an eye 30. The collimated light delivered through handpiece 10 and out of distal optical fiber 20 is generated by light source 12 and delivered to illuminate the retina 28 by means of fiber optic cable 14 and coupling system 32. Distal optical fiber 20 spreads the light beam delivered from light source 12 over a wider area of the retina than prior art probes.

FIGURE 13 provides another view of an endo-illuminator according to the teachings of this invention showing more clearly an embodiment of adjusting means 40. In this embodiment, adjusting means 40 comprises a slide button, as known to those skilled in the art. Activation of adjusting means 40 on handpiece 10 by, for example, a gentle and reversible sliding action, can cause the distal optical fiber 20 / proximal optical fiber 13 / tapered section 26/126/226 assembly to move laterally away from or towards the distal end of stem 16 by an amount determined and adjusted by sliding adjusting means 40. Thus, the angle of illumination and the amount of illumination provided by the illuminator probe to illuminate the surgical field (e.g., the retina 28 of an eye 30) can be easily adjusted within its limits by a surgeon using adjusting means 40. In this way, a surgeon can adjust the amount of light spread over a surgical field as desired to optimize the viewing field while minimizing glare. The adjusting means 40 of handpiece 10 can be any adjusting means known to those familiar with the art.

In one embodiment of the endo-illuminator of the present invention, a simple mechanical locking mechanism, as known to those skilled in the art, can permit the linear position of the distal optical fiber 20 / proximal optical fiber 13 / tapered section 26/126/226 assembly to be fixed, until released and/or re-adjusted by the user via the adjusting means 40. Thus, the pattern of light 32 emanating from the distal end of stem 16 will illuminate an area over a solid angle θ, the angle θ being continuously adjustable by a user (e.g., a surgeon) via the adjusting means 40 of handpiece 10.

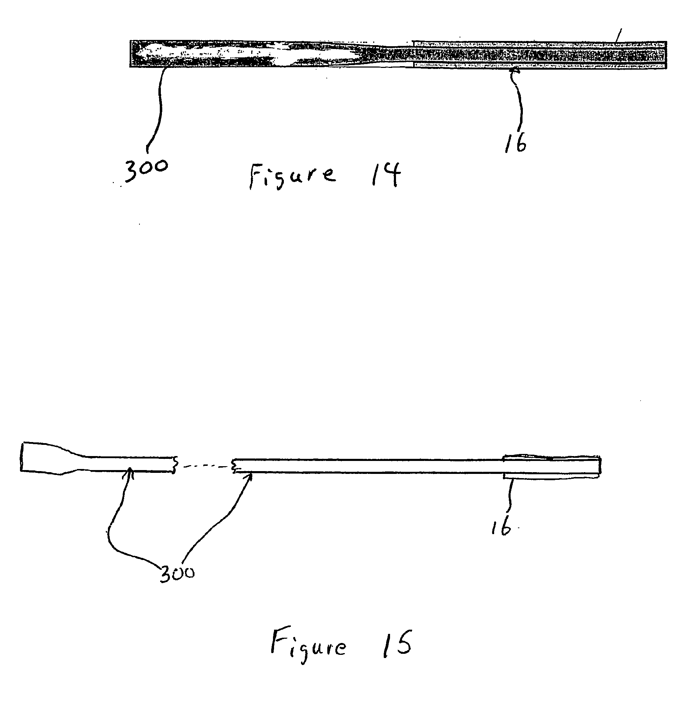

Other embodiments of the high throughput endo-illuminator of the present invention can comprise a single contiguous optical fiber 300 having a tapered section 26, in accordance with the teachings of this invention, in place of a separate proximal optical fiber 13 and a separate distal optical fiber 20. In such embodiments, the contiguous optical fiber 300 can be a smaller gauge (e.g., 20 gauge compatible), high NA optical fiber having a tapered section 26 near its distal end or, alternatively, a larger gauge (e.g., 25 gauge compatible), high-NA optical fiber having a tapered section 26 near its proximal end. In any of these embodiments, the NA of the contiguous optical fiber 300 should be higher throughout the length of contiguous optical fiber 300 than the NA of the light beam as it is transmitted along the contiguous optical fiber 300. FIGUREs 14 and 15 show exemplary embodiments of a contiguous optical fiber endo-illuminator in accordance with this invention. Contiguous optical fiber 300 can be produced by any of the methods described herein, such as stretching, belling, molding or any combination thereof.

Although the present invention has been described in detail herein with reference to the illustrated embodiments, it should be understood that the description is by way of example only and is not to be construed in a limiting sense. It is to be further understood, therefore, that numerous changes in the details of the embodiments of this invention and additional embodiments of this invention will be apparent to, and may be made by, persons of ordinary skill in the art having reference to this description. It is contemplated that all such changes and additional embodiments are within the scope of this invention as claimed below. Thus, while the present invention has been described in particular reference to the general area of ophthalmic surgery, the teachings contained herein apply equally wherever it is desirous to provide a illumination with higher gauge endo-illuminator.

## Claims

1. A high throughput endo-illuminator, comprising:
a first proximal optical fiber (13), optically coupled to a light source (12) and operable to transmit a light beam received from the light source; and further comprising
a second, distal optical fiber (20) distinct from the first proximal optical fiber, optically coupled to a distal end of the proximal optical fiber, for receiving the light beam and emitting the light beam to illuminate a surgical site, wherein the distal optical fiber comprises a tapered section (26) having a proximal-end diameter larger than a distal-end diameter;
a handpiece (10), operably coupled to the distal optical fiber; and
a cannula (16), operably coupled to the handpiece, for housing and directing the distal optical fiber.

2. The endo-illuminator of Claim 1, wherein the tapered section's (26) proximal end diameter is the same as the diameter of the proximal optical fiber (13).

3. The endo-illuminator of Claim 2, wherein the tapered section's (26) proximal end diameter is a 20 gauge compatible diameter and wherein the tapered section's distal end diameter is a 25 gauge compatible diameter.

4. The endo-illuminator of Claim 1, wherein the proximal optical fiber (13) is a 20 gauge compatible optical fiber, the cannula (16) is a 25 gauge inner diameter cannula and the distal optical fiber (20) has a 20 gauge compatible proximal-end diameter and a 25 gauge compatible distal-end diameter.

5. The endo-illuminator of Claim 1, wherein the proximal optical fiber (13) has a numerical aperture ("NA") of approximately .5 and the distal optical fiber has an NA greater than .5.

6. The endo-illuminator of Claim 1, wherein the proximal optical fiber (13) has an NA equal to or greater than the NA of the light source beam and wherein the distal optical fiber (20) has an NA greater than the proximal optical fiber and greater than the light source beam at any point in the distal optical fiber.

7. The endo-illuminator of Claim 1, wherein the cannula (16), the distal optical fiber (20) and the handpiece (10) are fabricated from biocompatible materials.

8. The endo-illuminator Claim 1, further comprising an SMA optical fiber connector to optically couple the proximal optical fiber (13) to the light source (12).

9. The endo-illuminator of Claim 1, wherein the distal optical fiber (20) is operably coupled to the handpiece (10) to enable linear displacement of the distal optical fiber within the cannula (16).

10. The endo-illuminator of Claim 9, further comprising a means (40) for adjusting the linear displacement of the distal optical fiber (20).

11. The endo-illuminator of Claim 10, wherein the means (40) for adjusting comprise a push/pull mechanism.

12. The endo-illuminator of Claim 11, wherein the amount of linear displacement of the distal optical fiber (20) determines an angle of illumination and an amount of illumination provided by the distal optical fiber element to illuminate the surgical site.

13. The endo-illuminator of Claim 1, wherein the light beam comprises a beam of relatively incoherent light.

14. The endo-illuminator of Claim 1, wherein the light source (12) is a xenon light source.

15. The endo-illuminator of Claim 1, wherein the proximal optical fiber (13) and the distal optical fiber (20) are optically coupled using an optical adhesive.

16. A high throughput endo-illumination surgical system (2) comprising:
a light source (12) for providing a light beam in combination with the endoilluminator of any one of claims 1 to 15.

## Patentansprüche

1. Endoilluminator mit hohem Durchsatz, der aufweist:
einen ersten, proximalen Lichtwellenleiter (13), der optisch an eine Lichtquelle (12) gekoppelt und so betreibbar ist, dass er einen von der Lichtquelle empfangenen Lichtstrahl überträgt; und der des Weiteren aufweist:
einen zweiten, distalen Lichtwellenleiter (20), der sich von dem ersten, proximalen Lichtwellenleiter unterscheidet, und der optisch an ein distales Ende des proximalen Lichtwellenleiters gekoppelt ist, um den Lichtstrahl zu empfangen und den Lichtstrahl auszusenden, um eine Operationsstelle zu beleuchten, wobei der distale Lichtwellenleiter einen konisch zulaufenden Abschnitt (26) aufweist, dessen Durchmesser am proximalen Ende größer ist, als der Durchmesser am distalen Ende;
ein Handstück (10), das betriebsbereit an den distalen Lichtwellenleiter gekoppelt ist; und
eine Kanüle (16), die betriebsbereit an das Handstück gekoppelt ist, um den distalen Lichtwellenleiter aufzunehmen und zu führen.

2. Endoilluminator nach Anspruch 1, wobei der Durchmesser des proximalen Endes des konisch zulaufenden Abschnitts (26) gleich dem Durchmesser des proximalen Lichtwellenleiters ist.

3. Endoilluminator nach Anspruch 2, wobei der Durchmesser des proximalen Endes des konisch zulaufenden Abschnitts (26) ein 20 Gauge (0,81 mm) - kompatibler Durchmesser ist, und wobei der Durchmesser des distalen Endes des konisch zulaufenden Abschnitts ein 25 Gauge (0,45 mm) -kompatibler Durchmesser ist.

4. Endoilluminator nach Anspruch 1, wobei der proximale Lichtwellenleiter (13) ein 20 Gauge (0,81 mm) -kompatibler Lichtwellenleiter ist, die Kanüle (16) eine Kanüle mit einem Innendurchmesser von 25 Gauge (0,45 mm) ist, und der distale Lichtwellenleiter (20) einen 20 Gauge (0,81 mm) -kompatiblen Durchmesser des proximalen Endes und einen 25 Gauge (0,45 mm) - kompatiblen Durchmesser des distalen Endes hat.

5. Endoilluminator nach Anspruch 1, wobei der proximale Lichtwellenleiter (13) eine numerische Apertur von ca. 0,5 hat und der distale Lichtwellenleiter eine numerische Apertur hat, die größer ist als 0,5.

6. Endoilluminator nach Anspruch 1, wobei der proximale Lichtwellenleiter (13) eine numerische Apertur größer gleich der numerischen Apertur des Lichtquellenstrahls hat, und wobei der distale Lichtwellenleiter (20) an jedem Punkt in dem distalen Lichtwellenleiter eine numerische Apertur hat, die größer ist, als die des proximalen Lichtwellenleiters und größer, als die des Lichtquellenstrahls.

7. Endoilluminator nach Anspruch 1, wobei die Kanüle (16), der distale Lichtwellenleiter (20) und das Handstück (10) aus Biomaterialien hergestellt sind.

8. Endoilluminator nach Anspruch 1, der des Weiteren einen Lichtwellenleiter-SMA-Stecker aufweist, um den proximalen Lichtwellenleiter (13) optisch an die Lichtquelle (12) zu koppeln.

9. Endoilluminator nach Anspruch 1, wobei der distale Lichtwellenleiter (20) betriebsbereit an das Handstück (10) gekoppelt ist, um ein lineares Verschieben des distalen Lichtwellenleiters in der Kanüle (16) zu ermöglichen.

10. Endoilluminator nach Anspruch 9, der des Weiteren eine Einrichtung (40) zum Einstellen der linearen Verschiebung des distalen Lichtwellenleiters (20) aufweist.

11. Endoilluminator nach Anspruch 10, wobei die Einrichtung (40) zum Einstellen einen Druck-Zug-Mechanismus aufweist.

12. Endoilluminator nach Anspruch 11, wobei der Betrag der linearen Verschiebung des distalen Lichtwellenleiters (20) den Beleuchtungswinkel und den Grad der Beleuchtung bestimmt, der von dem distalen Lichtwellenleiterelement bereitgestellt wird, um die Operationsstelle zu beleuchten.

13. Endoilluminator nach Anspruch 1, wobei der Lichtstrahl einen Strahl von relativ inkohärentem Licht aufweist.

14. Endoilluminator nach Anspruch 1, wobei die Lichtquelle (12) eine Xenonlichtquelle ist.

15. Endoilluminator nach Anspruch 1, wobei der proximale Lichtwellenleiter (13) und der distale Lichtwellenleiter (20) mittels eines optischen Klebers optisch verbunden sind.

16. Chirurgisches Endoilluminationssystem mit hohem Durchsatz (2), das aufweist:
eine Lichtquelle (2) zum Bereitstellen eines Lichtstrahls in Kombination mit dem Endoilluminator nach einem der Ansprüche 1 bis 15.

## Revendications

1. Endo-illuminateur à haut rendement, comprenant :
une première fibre optique proximale (13), couplée optiquement à une source de lumière (12) et pouvant être utilisée pour transmettre un faisceau de lumière reçu de la source de lumière ; et comprenant en outre :
une deuxième fibre optique distale (20) distincte de la première fibre optique proximale, couplée optiquement à une extrémité distale de la fibre optique proximale, pour recevoir le faisceau de lumière et émettre le faisceau de lumière pour éclairer un site chirurgical, dans lequel la fibre optique distale comprend une section effilée (26) ayant un diamètre d'extrémité proximale supérieur à un diamètre d'extrémité distale ;
une pièce à main (10), couplée fonctionnellement à la fibre optique distale ; et
une canule (16), couplée fonctionnellement à la pièce à main, pour loger et diriger la fibre optique distale.

2. Endo-illuminateur selon la revendication 1, dans lequel le diamètre d'extrémité proximale de la section effilée (26) est identique au diamètre de la fibre optique proximale (13).

3. Endo-illuminateur selon la revendication 2, dans lequel le diamètre d'extrémité proximale de la section effilée (26) est un diamètre compatible avec une jauge 20, et dans lequel le diamètre d'extrémité distale de la section effilée est un diamètre compatible avec une jauge 25.

4. Endo-illuminateur selon la revendication 1, dans lequel la fibre optique proximale (13) est une fibre optique compatible avec une jauge 20, la canule (16) est une canule à diamètre intérieur de jauge 25 et la fibre optique distale (20) a un diamètre d'extrémité proximale compatible avec une jauge 20 et un diamètre d'extrémité distale compatible avec une jauge 25.

5. Endo-illuminateur selon la revendication 1, dans lequel la fibre optique proximale (13) a une ouverture numérique (« NA ») d'environ 0,5 et la fibre optique distale a une ouverture NA supérieure à 0,5.

6. Endo-illuminateur selon la revendication 1, dans lequel la fibre optique proximale (13) a une ouverture NA égale ou supérieure à l'ouverture NA du faisceau de source de lumière, et dans lequel la fibre optique distale (20) a une ouverture NA supérieure à celle de la fibre optique proximale et supérieure à celle du faisceau de source de lumière en n'importe quel point de la fibre optique distale.

7. Endo-illuminateur selon la revendication 1, dans lequel la canule (16), la fibre optique distale (20) et la pièce à main (10) sont fabriquées à partir de matériaux biocompatibles.

8. Endo-illuminateur selon la revendication 1, comprenant en outre un connecteur de fibre optique SMA pour coupler optiquement la fibre optique proximale (13) à la source de lumière (12).

9. Endo-illuminateur selon la revendication 1, dans lequel la fibre optique distale (20) est couplée fonctionnellement à la pièce à main (10) pour permettre un déplacement linéaire de la fibre optique distale dans la canule (16).

10. Endo-illuminateur selon la revendication 9, comprenant en outre des moyens (40) pour ajuster le déplacement linéaire de la fibre optique distale (20).

11. Endo-illuminateur selon la revendication 10, dans lequel les moyens (40) pour ajuster comprennent un mécanisme de poussée/traction.

12. Endo-illuminateur selon la revendication 11, dans lequel la quantité de déplacement linéaire de la fibre optique distale (20) détermine un angle d'éclairage et une quantité d'éclairage fournie par l'élément de fibre optique distale pour éclairer le site chirurgical.

13. Endo-illuminateur selon la revendication 1, dans lequel le faisceau de lumière comprend un faisceau de lumière relativement non cohérente.

14. Endo-illuminateur selon la revendication 1, dans lequel la source de lumière (12) est une source de lumière au xénon.

15. Endo-illuminateur selon la revendication 1, dans lequel la fibre optique proximale (13) et la fibre optique distale (20) sont couplées optiquement en utilisant un adhésif optique.

16. Système chirurgical d'endo-éclairage à haut rendement (2), comprenant :
une source de lumière (12) pour fournir un faisceau de lumière en combinaison avec l'endo-illuminateur selon l'une quelconque des revendications 1 à 15.
